# FASCICULE DE BREVET EUROPEEN

(11) **EP 3 863 561 B1**
(45) Date de publication et mention de la délivrance du brevet: **07.09.2022**
(21) Numéro de dépôt: 19783057.3
(22) Date de dépôt: 09.10.2019
(51) Int. Cl.: A61F 2/07, A61F 2/06

(54) **IMPLANT AORTIQUE DE TYPE STENT, ET ENSEMBLE FORMÉ DE DEUX TELS IMPLANTS**
STENTARTIGES AORTENIMPLANTAT UND AUS ZWEI SOLCHEN IMPLANTATEN GEBILDETE ANORDNUNG
STENT-TYPE AORTIC IMPLANT AND ASSEMBLY FORMED BY TWO SUCH IMPLANTS

(30) Priorité: 12.10.2018 FR 1871165
(43) Date de publication de la demande: 18.08.2021
(73) Titulaire: Canaud, Ludovic, 34000 Montpellier (FR); Gandet, Thomas, 34090 Montpellier (FR)
(72) Inventeur: Canaud, Ludovic, 34000 Montpellier (FR); Gandet, Thomas, 34090 Montpellier (FR)
(74) Mandataire: LLR
(86) Numéro de dépôt international: PCT/EP2019/077390
(87) Numéro de publication internationale: WO 2020/074598

(56) Documents cités:
- WO-A1-2014/141232
- WO-A2-2007/028086

## Description

La présente invention concerne un implant aortique de type stent.

Comme on le sait, l'aorte se courbe sur 180° peu après le cœur, et l'on distingue la "crosse" de l'aorte, c'est-à-dire le sommet de la portion courbe de l'aorte, l'aorte dite "ascendante", c'est-à-dire la partie de cette portion courbe située entre le cœur et la crosse, et l'aorte dite "descendante", c'est-à-dire la partie de cette portion courbe située entre la crosse et la fin de la portion courbe. De cette crosse aortique naissent les artères brachio-céphalique, carotide commune gauche et sous-clavière gauche permettant l'apport de sang au niveau du cerveau.

Il existe plusieurs types de lésions intéressant la crosse aortique : les anévrysmes dégénératifs, qui consistent en une augmentation du calibre de l'aorte d'au moins de deux fois le calibre normal, les dissections aortiques, qui consistent en une déchirure de la paroi de l'aorte selon l'épaisseur de cette paroi, les ruptures traumatiques de la crosse aortique, les ulcères aortiques, qui sont des perforations liées à la présence d'athérome. Le risque engendré par tous ces types de lésions est celui d'une rupture de la crosse aortique avec pour conséquence une hémorragie interne et un décès inéluctable. Toutes ces lésions constituent une indication chirurgicale si le diamètre de l'aorte dépasse les 5,5 cm ou si elles sont symptomatiques.

Il est connu de traiter ces types de lésions au moyen d'un implant aortique de type stent, comprenant une armature déployable en fil métallique et une membrane recouvrant cette armature. Un implant actuellement utilisé pour ce traitement est en fait un implant conçu pour le traitement d'affections de l'aorte descendante ou de l'aorte thoracique (au-delà de l'aorte descendante), ayant une longueur minimale de dix centimètres. L'implant, placé à l'état contracté dans une gaine, est amené par voie endovasculaire jusqu'au niveau de la crosse aortique et est déployé de façon à recouvrir la lésion aortique. Au préalable, il est nécessaire de procéder à des détournements des artères précitées, dont les débouchés dans l'aorte vont être recouverts par l'implant ; ces détournements se font sous la forme de pontages, dont la réalisation implique l'ouverture de la cage thoracique et l'arrêt partiel de la circulation dans l'aorte ; ces opérations sont longues et complexes, et la nécessité de les mettre en œuvre est un fort inconvénient de cet implant.

D'autres implants, réalisés sur mesure, ont été développés. Un implant de ce type présente une partie aortique à laquelle deux branches sont raccordées ; ces branches sont destinées à être mises en place dans deux des artères à destinée cérébrale précitées ; un maintien de l'afflux sanguin dans la dernière des trois de ces artères est réalisé par un pontage chirurgical. Cette technique permet un traitement qui est pratiquement complètement endovasculaire ; elle présente cependant de nombreux désavantages : premièrement, la nécessité d'un pontage pour maintenir la perfusion d'une des trois artères précitées étant donné que l'implant aortique ne permet que l'utilisation de deux branches ; deuxièmement, les branches sont insérées à partir des débouchés de deux des artères cérébrales, ce qui implique un arrêt temporaire de la perfusion cérébrale et une manipulation de ces artères, sources d'un taux élevé d'accident vasculaire cérébral ; troisièmement, le délai nécessaire à la fabrication d'un tel implant sur mesure est une contrainte notable et ne permet pas l'utilisation d'un tel implant dans une situation d'urgence ou pour les patients qui sont asymptomatiques mais qui présentent une lésion aortique volumineuse à haut risque de rupture.

Par ailleurs, la publication de demande de brevet N° EP 3 017 790 A2 décrit un implant ayant les caractéristiques définies par la partie pré-caractérisante de la revendication 1 annexée. L'implant selon ce document antérieur ne remédie que très partiellement aux inconvénients précités. En effet, il ne permet pas de traiter un certain nombre des affections susceptibles d'exister au niveau de la partie ascendante de l'aorte et au niveau de la partie proximale de la crosse de l'aorte. L'implant selon ce document antérieur est par ailleurs spécifiquement destiné à être mis en place de façon antérograde depuis le TABC (Tronc artériel brachio-céphalique), raison pour laquelle il présente une encoche proximale ; cette encoche ne confère pas à l'implant l'étanchéité requise au niveau des débouchés des artères brachio-céphalique et carotide commune gauche et ne permet pas la perfusion du TABC et de la carotide gauche dans les meilleures conditions. L'ancrage de l'implant est également susceptible d'amélioration. En outre, le positionnement de la partie aortique de l'implant par rapport à l'artère sous-clavière gauche est imprécis.

La publication de la demande de brevet N° WO 2014/141232 A1 divulgue un implant ne permettant pas non plus de parfaitement remédier aux inconvénients précités.

Ce document divulgue les caractéristiques mentionnées dans la partie pré-caractérisante de la revendication 1 annexée.

Les caractéristiques constituant l'invention sont définies dans la partie caractérisante de la revendication 1.

La présente invention a pour objectif de remédier à l'ensemble de ces inconvénients.

En pratique, ladite partie aortique, dans son état de contraction, est engagée dans l'aorte et est positionnée de telle sorte qu'un ou plusieurs marqueurs radio-opaques présents sur la partie aortique soient positionnés sensiblement en regard du débouché de ladite première artère ; la partie aortique est alors déployée partiellement dans l'aorte jusqu'à sa portion qui comprend ladite première ouverture ; ladite branche est, alors qu'elle est dans son état de contraction, engagée dans l'artère sous-clavière gauche puis au travers de ladite première ouverture et est ensuite déployée dans cette première ouverture puis dans l'artère sous-clavière gauche, ce déploiement permettant de lier cette branche à la partie aortique de l'implant et à cette artère ; le renfort que comprend le bord de ladite membrane qui délimite ladite première ouverture permet à ce bord de supporter l'effort résultant du déploiement de ladite branche sans risque de déchirure de la membrane ; l'implant est ainsi positionné à la fois longitudinalement et angulairement dans l'aorte. Le reste de ladite partie aortique est alors déployé pour terminer la mise en place de l'implant. Ladite deuxième ouverture est alors parfaitement positionnée en regard des débouchés des deux artères autres que l'artère sous-clavière gauche, et la partie aortique est parfaitement appliquée autour de ces débouchés compte tenu de la présence desdits segments des structures en V le long de la majorité des bords de la membrane qui délimitent cette deuxième ouverture. Il en résulte la possibilité de réaliser la perfusion du TABC (Tronc artériel brachio-céphalique) et de l'artère carotide gauche dans les meilleures conditions. De plus, ladite deuxième structure en V, libérée de toute membrane, vient se déployer dans le TABC et permet un meilleur ancrage de l'implant ainsi qu'une meilleure conformabilité de ladite deuxième ouverture. En outre, ladite partie aortique, par le fait qu'elle forme ladite deuxième ouverture, présente par conséquent une partie proximale apte à s'étendre non seulement au niveau de la partie proximale de la crosse de l'aorte mais également dans la partie ascendante de cette aorte, ce qui permet à l'implant selon l'invention d'être à même de traiter des pathologies s'étendant en ces emplacements.

L'implant selon l'invention est ainsi conçu de façon à présenter une partie aortique et une branche formant deux sous-ensembles séparés, et à présenter une première ouverture ayant une dimension adaptée pour réaliser une liaison de ladite branche à ladite partie aortique lorsque cette branche est dans son état de déploiement. Cette liaison permet un positionnement précis de ladite partie aortique dans l'aorte et donc un positionnement de ladite deuxième ouverture sensiblement en regard des débouchés des deux artères autres que l'artère sous-clavière gauche. L'implant, une fois mis en place, ne réalise donc aucun recouvrement des débouchés des trois artères précitées et n'implique dès lors aucune mise en œuvre d'un ou plusieurs pontages. De plus, la mise en place de cet implant n'implique pas de manipulation de l'artère sous-clavière gauche ou de l'une et/ou l'autre desdites deux autres artères, et élimine ainsi le risque d'accident vasculaire cérébral. Cet implant ne nécessite par ailleurs pas de délai de fabrication notable, et n'implique donc pas de contrainte notable de ce point de vue.

De préférence, ledit renfort est radio-opaque et flexible, et ladite première ouverture est située à proximité du coude distal de ladite première structure en V formée par ledit troisième anneau.

Ladite première ouverture (10) est donc située entre les segments de la structure en V adjacente du quatrième anneau ; dans l'état de contraction de l'armature déployable, ces segments sont rapprochés, contractant la portion de membrane s'étendant entre eux et amenant donc le renfort de ladite première ouverture, dans un état de contraction ; dans cet état de contraction, ce renfort forme un repère allongé sur une image d'imagerie médicale, et constitue donc un élément fiable de repérage de l'orientation de ladite partie aortique, ce qui permet de définir précisément l'emplacement de la première ouverture le long de ladite partie aortique.

De préférence, les coudes distaux de l'un desdits premier à troisième anneaux sont situés à une distance allant de 0 à 10 mm d'un plan transversal à la partie aortique passant par les coudes proximaux de l'anneau distalement adjacent.

Lesdits premier à quatrième anneaux pourraient présenter, à l'état déployé, un diamètre égal à celui que présente la membrane à l'état déployé ; de préférence, toutefois, ces anneaux présentent, à l'état déployé, un diamètre supérieur à celui que présente la membrane à l'état déployé.

Le tissu que forme la membrane est ainsi bien tendu au niveau des bords qui délimitent ladite deuxième ouverture et un effet de fronce est évité. Une bonne adhérence de la membrane autour des débouchés des deux artères précitées est obtenue, et des fuites sont évitées.

De préférence, les anneaux ont, à l'état déployé, un diamètre qui est de 1 à 30% supérieur au diamètre que présente la membrane à l'état déployée, et qui est de préférence de l'ordre de 20% supérieur à ce diamètre.

Ladite branche pourrait être reliée à ladite partie aortique par simple prise d'appui de cette branche contre ledit renfort délimitant ladite première ouverture ; de préférence, toutefois, l'extrémité de ladite branche destinée à être engagée au travers de ladite première ouverture présente, dans l'état de déploiement de ladite branche, une collerette ou une portion de forme évasée ayant un plus grand diamètre que celui de ladite première ouverture, cette collerette ou cette portion de forme évasée étant ainsi apte à prendre appui contre ladite partie aortique de l'implant, au niveau de la face interne de cette dernière.

Une liaison fiable de ladite branche et de ladite partie aortique est ainsi obtenue.

Ladite première ouverture peut avoir un diamètre allant de 5 à 11 mm ; ce diamètre est de préférence de 8 mm.

Ladite deuxième ouverture peut avoir une dimension dans la direction longitudinale de ladite partie aortique allant de 20 à 40 mm et une dimension dans la direction circonférentielle de cette partie aortique allant de 20 à 40 mm.

Ladite partie aortique comprend avantageusement un ou plusieurs marqueurs radio-opaques situés au voisinage de ladite deuxième ouverture, agencés de façon à permettre de visualiser le plan de cette deuxième ouverture sur une image d'imagerie médicale.

Ces marqueurs permettent ainsi de visualiser également le positionnement de cette deuxième ouverture.

L'invention concerne par ailleurs également un ensemble formé par deux implants aortiques de type stent tels que précités, cet ensemble comprenant :
- un premier implant dans lequel le bord de ladite première ouverture tourné vers le bord adjacent de ladite deuxième ouverture se trouve à une distance de ce bord adjacent allant de 4 à 8 mm, de préférence égale à 5 mm ; et
- un deuxième implant dans lequel le bord de ladite première ouverture tourné vers le bord adjacent de ladite deuxième ouverture se trouve à une distance de ce bord adjacent allant de 8 à 12 mm, de préférence égale à 10 mm.

Il apparaît que le premier implant permet de traiter environ 80 % des patients ; le deuxième implant est adapté au traitement des 20 % de patients restant.

L'invention sera bien comprise, et d'autres avantages et caractéristiques de celle-ci apparaîtront, en référence au dessin schématique annexé, qui représente, à titre d'exemple non limitatif, une forme de réalisation préférée de l'implant concerné.
La figure 1 est une vue de côté, selon une première direction, d'une partie aortique de cet implant ;
la figure 2 est une vue de ladite partie aortique de côté selon une deuxième direction, sensiblement perpendiculaire à ladite première direction ;
la figure 3 est une vue d'une aorte en coupe et de ladite partie aortique dans un état de contraction, au cours d'une première étape de mise en place de cette partie aortique dans cette aorte ;
la figure 4 est une vue similaire à la figure 3, ladite partie aortique étant partiellement déployée ; une branche latérale sous forme de stent, qui constitue l'implant avec ladite partie aortique, est engagée, dans un état de contraction, au travers de l'artère sous-clavière gauche ; et
la figure 5 est une vue similaire à la figure 4 de l'implant une fois mise en place, ladite partie aortique et ladite branche étant complètement déployées.

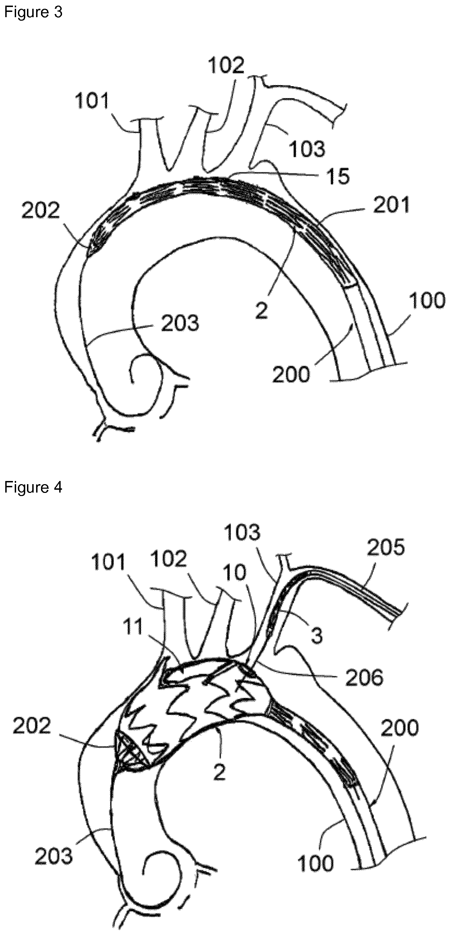

Les figures 3 à 5 représentent, de façon très schématique, vues en coupe dans un plan frontal, une aorte 100 et les artères brachio-céphalique 101, carotide commune gauche 102 et sous-clavière gauche 103 qui débouchent dans l'aorte 100 et permettent l'apport de sang au niveau du cerveau.

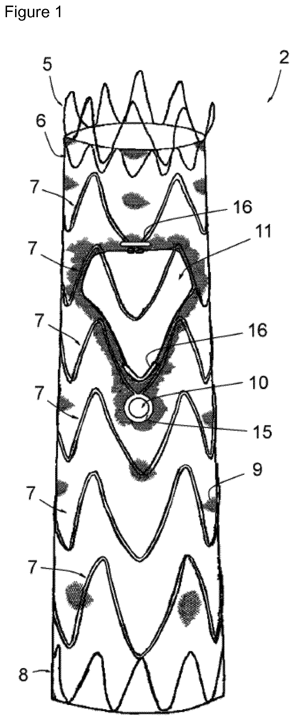

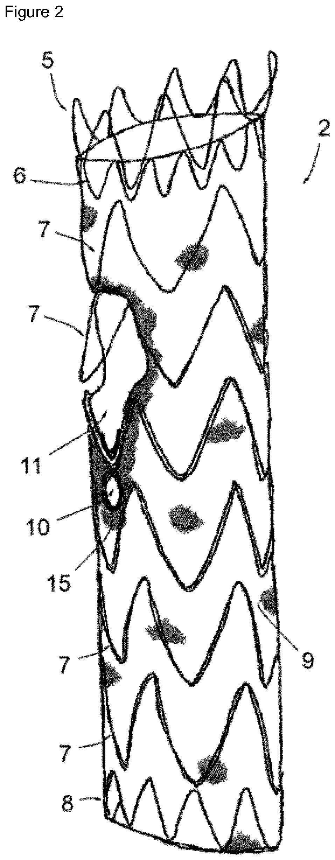

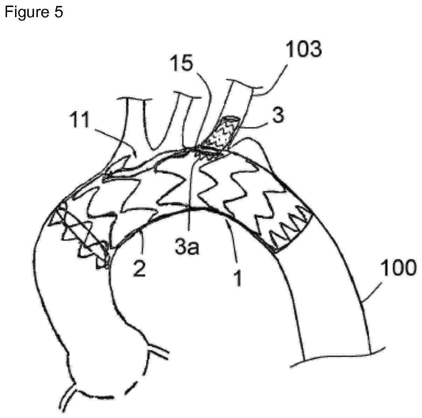

La figure 5 montre l'implant aortique 1 de type stent selon l'invention, qui est formé par la partie aortique 2 montrée sur les figures 1 et 2 et par la branche 3 déployée dans l'artère sous clavière gauche 103. Cette partie aortique 2 et cette branche 3 forment deux sous-ensembles séparés, aptes à être assemblés l'un à l'autre.

Comme cela est plus particulièrement visible sur les figures 1 et 2, la partie aortique 2 est de type stent, c'est-à-dire comprend une armature déployable 5, 6, 7, 8 en fil métallique et une membrane 9 recouvrant cette armature.

Par souci de clarté du dessin, la membrane 9 n'est que partiellement représentée ; elle est cependant montrée de façon substantiellement complète autour de deux ouvertures 10 et 11 qu'elle comprend, afin de bien faire apparaître ces deux ouvertures. Pour faire apparaître les différents éléments 5, 6, 7, 8 constituant ladite armature, la membrane 9 est fictivement représentée comme étant transparente mais, toujours par souci de clarté du dessin, les parties de ces éléments 5, 6, 7, 8 qui sont situées dans la moitié de la circonférence de la partie aortique 2 apparaissant en arrière-plan sur les figures ne sont pas représentées.

Dans l'exemple représenté, l'armature 5, 6, 7, 8 est formée, depuis son extrémité proximale vers son extrémité distale, par une série d'anneaux 5 à 8 dont chacun est formé par un fil métallique en ligne brisée. Comme cela est très classique, la direction proximale-distale est à considérer dans le sens de l'écoulement du sang au travers de l'aorte 100.

Cette structure en ligne brisée, bien connue en elle-même, permet à la partie aortique 2 de pouvoir prendre un état de contraction visible sur la figure 3 lorsque la partie aortique 2 est placée dans une gaine 201 que comprend le cathéter 200 d'insertion et de mise en place de cette partie aortique 2 ; cette même structure en ligne brisée permet également à la partie aortique 2 de pouvoir prendre, par mémoire de forme ou par déploiement au moyen d'un ballonnet, un état d'expansion visible sur la figure 5, dans lequel la partie aortique 2 et au contact de la paroi de l'aorte 100. Les anneaux 5 à 8 sont par ailleurs indépendants les uns des autres et sont reliés les uns aux autres uniquement au moyen de la membrane 9, ce qui permet que la partie aortique 2 soit suffisamment déformable transversalement pour pouvoir suivre la courbure de l'aorte 100.

L'armature 5, 6, 7, 8 peut notamment être réalisée en alliage de nickel et de titane, et la membrane 9 peut notamment être en polyester tissé ou en polytétrafluoroéthylène expansé.

Dans l'exemple représenté, un anneau proximal 5 d'extrémité présente, entre les coudes que forme la ligne brisée que dessine cet anneau, des segments ayant des longueurs supérieures aux longueurs des segments homologues que présente un deuxième anneau proximal 6 d'extrémité ; ces segments de l'anneau 5 ont des longueurs inférieures aux longueurs des segments d'anneaux 7. L'armature est complétée par un anneau distal d'extrémité 8 dont les segments sont sensiblement de mêmes longueurs que ceux de l'anneau proximal 6, ou sont légèrement plus grands.

Les anneaux 7 qui sont dénommés ci-après "centraux" ou "de corps" de la partie aortique pour les distinguer des anneaux d'extrémité 5, 6 et 8.

L'anneau 5 n'est que très partiellement recouvert par la membrane 9 et permet ainsi un maintien de l'extrémité proximale la partie aortique 2 au cours de la mise en place de cette partie aortique 2, au moyen d'une pièce proximale de maintien 202, déployable, portée par un fil-guide 203 que comprend le cathéter 200 d'introduction de la partie aortique 2 dans l'aorte 100. Un tel cathéter d'introduction est de type classique, bien connu en lui-même, et n'est donc pas particulièrement détaillé. L'anneau 5 permet également, de par l'absence de membrane 9 autour de lui, un ancrage renforcé de l'extrémité proximale de la partie aortique 2 à l'aorte 100 ; les structures en V inversé qu'il forme peuvent être légèrement défléchies radialement vers l'extérieur, comme visible sur la figure 2, afin de favoriser l'ancrage de cet anneau 5 à la paroi de l'aorte 100.

L'anneau 6 permet assurer le maintien de l'extrémité proximale de la membrane 9 en position de déploiement.

Les anneaux centraux 7 permettent de déployer la membrane 9 et de la maintenir contre la paroi de l'aorte 100 ; ils participent au maintien longitudinal de la partie aortique 2 dans l'aorte 100. Chacun de ces anneaux centraux 7 est formé par un fil métallique en ligne brisée, et présente ainsi des segments rectilignes séparés par des coudes ; deux segments consécutifs et un coude s'étendant entre ces segments définissent une structure dénommée plus loin "structure en V". Chaque anneau 7 forme cinq ou six structure en V. Ces anneaux centraux 7, pour être différenciés les uns par rapport aux autres dans la description faite plus loin, seront dénommés respectivement premier, deuxième, troisième, quatrième anneaux, et ainsi de suite, depuis l'extrémité proximale de la partie aortique 2 vers l'extrémité distale de cette même partie, donc depuis les parties supérieures des figures 1 et 2 en descendant vers la partie inférieure de ces mêmes figures.

Les anneaux 7 sont consécutifs les uns des autres, ou immédiatement consécutifs les uns des autres, en ce sens que les coudes distaux de l'un desdits premier à troisième anneaux 7 sont situés à une distance d'un plan transversal à la partie aortique 2 séparant ces coudes distaux des coudes proximaux de l'anneau 7 adjacent, qui est égale à 0 mm dans l'exemple représenté, ou qui peut, sinon, être de 1 à 10 mm.

L'anneau 8 permet quant à lui de maintenir l'extrémité distale de la membrane 9 en position de déploiement.

Les deux ouvertures 10 et 11 sont aménagées sur un côté de la partie aortique 2, au travers de la membrane 9, en succession l'une de l'autre mais séparées l'une de l'autre.

L'ouverture 10 est circulaire et est délimitée par un renfort périphérique 15 cousu à la membrane 9. Ce renfort 15 renforce l'ensemble de la périphérie de l'ouverture 10 tout en étant flexible, c'est-à-dire ne faisant pas obstacle à une contraction de l'ouverture 10 lorsque la membrane est contractée radialement, à l'état de contraction de la partie aortique 2. Le renfort 15 comporte en outre un produit radio-opaque permettant sa visibilité sur une image d'imagerie médicale.

Le diamètre interne de l'ouverture 10 est de 8 mm, et est légèrement inférieur à la section transversale que présente la branche 3 dans son état de déploiement dans l'artère 103.

Dans l'exemple représenté, l'ouverture 10 est située à proximité du coude distal d'une première structure en V formée par le troisième anneau 7 à partir de l'extrémité proximale de la partie aortique 2, cette structure en V ayant ses deux segments qui divergent en direction de l'extrémité proximale de cette partie aortique 2 ; le centre de l'ouverture 10 est sensiblement situé sur l'axe de symétrie de cette première structure en V ; ce centre est situé sensiblement à mi-longueur de la membrane 9.

L'ouverture 11 est, dans l'exemple représenté, aménagée au-dessus d'une deuxième structure en V formée par le deuxième anneau 7 à partir de l'extrémité proximale de la partie aortique 2, cette deuxième structure ayant ses deux segments qui divergent en direction de l'extrémité proximale de la partie aortique 2 et étant située sur ledit axe de symétrie. En longueur, l'ouverture 11 s'étend entre :
- un bord proximal transversal situé au niveau du coude distal que forme une troisième structure en V formée, sur ledit axe de symétrie, par le premier anneau 7 ;
- des premiers bords latéraux, divergents dans la direction distale, qui s'étendent entre les segments situés de part et d'autre des segments formant ladite deuxième structure en V, et donc consécutifs à ces segments sur la circonférence dudit deuxième anneau 7 ;
- des deuxièmes bords latéraux, convergeant dans la direction distale, qui s'étendent le long des segments de ladite première structure en V du troisième anneau 7 ; et
- le coude distal formé par ladite première structure en V.

L'ouverture 11 présente ainsi une forme "en poire", dont la plus grande largeur est dirigée du côté proximal et dont la pointe est dirigée du côté distal.

Les bords de la membrane 9 qui délimitent l'ouverture 11 sont reliés, depuis le côté proximal et en direction du côté distal de la partie aortique 2, au coude distal formé par ladite troisième structure en V, auxdits segments extérieures à ladite deuxième structure en V, auxdits segments de ladite première structure en V et audit coude distal formé par cette première structure en V. Ces bords de la membrane 9 sont ainsi parfaitement reliés aux anneaux 7 concernés et sont donc parfaitement renforcés et maintenus par ces anneaux.

La membrane 9 comporte en outre des inserts 16 en matériau radio-opaque, dont un, dans l'exemple représenté, est fixé au niveau du coude distal de ladite troisième structure en V et l'autre est fixé au niveau du coude distal de ladite première structure en V. Ces inserts 16 permettent de visualiser la position de l'ouverture 11 sur une image d'imagerie médicale.

La branche 3 est de type stent, étant formée par une armature déployable. Comme cela apparaît sur la figure 4, cette branche 3 est apte, dans un état de contraction, à être engagée dans l'artère sous-clavière gauche 103 au moyen d'un cathéter 205 ; comme visible sur la figure 5, la branche 3 est apte, dans un état d'expansion, à être engagée au travers de l'ouverture 10 et à prendre appui contre la paroi de l'artère sous-clavière gauche 103.

L'extrémité de la branche 3 destinée à être engagée au travers de l'ouverture 10 présente, dans l'état de déploiement de la branche 3, une collerette ou une portion 3a de forme évasée ayant un plus grand diamètre que celui de l'ouverture 10. Cette collerette ou cette portion 3a de forme évasée est ainsi apte à prendre appui contre la membrane 9, au niveau de la face interne de cette dernière et au niveau de l'anneau 15, comme visible sur la figure 5.

Les figures 3 à 5 illustrent trois étapes successives de la mise en place de l'implant 1 formé par la partie aortique 2 et par la branche 3.

En pratique, la partie aortique 2, maintenue dans son état de contraction par la gaine 201, est engagée dans l'aorte 100 par voie endovasculaire, en étant guidée par le fil-guide 203 ; ainsi que cela apparaît sur la figure 3, elle est positionnée de telle sorte que le renfort 15 soit placé sensiblement en regard du débouché de l'artère sous-clavière gauche 103. Ce renfort 15, tel qu'il est visible dans l'état de contraction de la membrane par imagerie médicale, forme un repère radio-opaque allongé qui constitue un élément fiable de repérage de l'orientation de ladite partie aortique 2 et permet de définir précisément l'emplacement de l'ouverture 10 le long de cette partie aortique 2.

La partie aortique 2 est alors déployée partiellement dans l'aorte 10 jusqu'à sa portion qui comprend l'ouverture 10, ce qui positionne l'ouverture 11 sensiblement au regard des débouchés des artères brachio-céphalique 101 et carotide commune gauche 102. Cette partie aortique, par le fait qu'elle forme cette ouverture 11, présente par conséquent une partie proximale apte à s'étendre non seulement au niveau de la partie proximale de la crosse de l'aorte mais également dans la partie ascendante de cette aorte, ce qui permet à l'implant d'être à même de traiter des pathologies s'étendant en ces emplacements.

L'ouverture 11 est, dans cette position, parfaitement positionnée en regard des débouchés des deux artères 101, 102, et la partie aortique 2 est parfaitement appliquée autour de ces débouchés compte tenu de la présence desdits segments des structures en V le long de la majorité des bords de la membrane 9 qui délimitent cette ouverture 11. Il en résulte la possibilité de réaliser la perfusion du TABC (Tronc artériel brachio-céphalique) et de l'artère carotide gauche dans les meilleures conditions. De plus, ladite deuxième structure en V, libérée de toute membrane, vient se déployer dans le TABC et permet un meilleur ancrage de l'implant ainsi qu'une meilleure conformabilité de l'ouverture 11.

Un fil-guide 206 est alors introduit dans l'artère sous-clavière gauche 103 puis au travers de l'ouverture 10, jusqu'à l'intérieur de la partie aortique 2, puis le cathéter 205, contenant la branche 3 maintenue à l'état contracté par une gaine, est utilisé pour amener, par coulissement sur ce fil-guide 206, la branche 3 au travers de cette artère 103, ainsi que le montre la figure 4.

Cette branche 3 est avancée jusqu'à ce que sa portion proximale soit engagée au travers de l'ouverture 10, puis la gaine du cathéter 205 est reculée de façon à initier le déploiement de la branche 3. La collerette ou portion 3a à forme évasée de l'extrémité de la branche 3 se déploie ainsi à l'intérieur de la partie aortique 2 et vient porter contre la face interne de la membrane 9, au niveau du renfort 15, réalisant une liaison de la branche 3 à la partie aortique 2. Le renfort 15 permet à la membrane 9 de supporter l'effort résultant du déploiement de la branche 3 sans risque de déchirure de cette membrane.

Le déploiement de la branche 3 est poursuivi jusqu'à une prise d'appui de cette branche 3 contre la paroi de l'artère sous-clavière gauche 103, puis le cathéter 205 est retiré.

L'implant 1 est ainsi positionné à la fois longitudinalement et angulairement dans l'aorte 100, par le déploiement de la branche 3 dans l'artère 103 et par la liaison de cette branche 3 à la partie aortique 2, et est parfaitement maintenu dans cette position.

L'anneau proximal 5 est ensuite libéré par la pièce 202 et le reste de la partie aortique 2 est déployé dans l'aorte descendante, ainsi que le montre la figure 5, sans risque de déplacement de l'implant 1.

L'invention fournit ainsi un implant aortique de type stent remédiant aux inconvénients des implants homologues de la technique antérieure, par le fait qu'il est apte à être précisément positionné dans l'aorte, et qu'il permet de positionner précisément des ouvertures ou fenêtres traversant sa paroi en regard des débouchés des artères brachio-céphalique, carotide commune gauche et sous-clavière gauche, donc en éliminant la nécessité de procéder à des pontages ; la mise en place de cet implant n'implique pas de manipulation desdites artères et élimine ainsi le risque d'accident vasculaire cérébral ; cet implant ne nécessite par ailleurs pas de délai de fabrication notable, et n'implique donc pas de contrainte notable de ce point de vue.

## Revendications

1. Implant aortique (1) de type stent, comprenant une partie aortique (2) et une branche (3) destinée à être placée dans l'artère sous-clavière gauche ; ladite partie aortique (2) comprend une armature déployable (5, 6, 7, 8) en fil métallique et une membrane (9) recouvrant cette armature ;
ladite armature déployable inclut des anneaux (7) indépendants les uns des autres et reliés les uns aux autres uniquement au moyen de la membrane (9), dont chacun est formé par un fil métallique en ligne brisée, chaque anneau présentant ainsi des segments rectilignes séparés par des coudes, deux segments consécutifs et un coude qui s'étend entre ces segments définissant une structure en "V" ; l'armature comprend des premier, deuxième, troisième et quatrième anneaux (7) comptés à partir de l'extrémité proximale de la partie aortique (2) ;
ladite branche (3) est formée par une armature déployable apte, dans un état de contraction, à être engagée dans l'artère sous-clavière gauche et apte, dans un état d'expansion, à prendre appui contre la paroi de cette artère ;
l'implant présente une ouverture (11), dite deuxième ouverture (11), dimensionnée, de manière à se trouver sensiblement en regard des débouchés des artères brachio-céphalique et carotide commune gauche après mise en place de l'implant ;
**caractérisé en ce que** :
ladite partie aortique (2) et ladite branche (3) sont formées par deux sous-ensembles séparés, aptes à être assemblés l'un à l'autre ;
ladite partie aortique (2) présente une première ouverture (10) aménagée au travers de ladite membrane (9), dimensionnée de façon à présenter une surface au moins égale, ou inférieure, à la section transversale que présente ladite branche (3) dans son état de déploiement dans l'artère sous-clavière gauche; ladite deuxième ouverture (11) est aménagée sur la partie aortique (2), au travers de ladite membrane (9), et est disposée par rapport à ladite première ouverture (10), et dimensionnée, de manière à se trouver sensiblement en regard du débouché de l'artère carotide commune gauche lorsque ladite première ouverture (10) se trouve sensiblement en regard du débouché de l'artère sous-clavière gauche ;
**caractérisé en ce que** :
- le centre de ladite première ouverture (10) est situé sur l'axe de symétrie d'une première structure en V formée par ledit troisième anneau (7), cette première structure en V ayant ses deux segments qui divergent en direction de l'extrémité proximale de ladite partie aortique (2) ; la première ouverture est délimitée par un bord présentant un renfort (15) qui s'étend sur l'ensemble de la périphérie de cette première ouverture (10) ;
- ladite deuxième ouverture (11) comprend une partie proximale aménagée au-dessus d'une deuxième structure en V formée par ledit deuxième anneau (7), cette deuxième structure ayant ses deux segments qui divergent en direction de l'extrémité proximale de la partie aortique (2) et étant située sur ledit axe de symétrie ; en longueur, ladite deuxième ouverture (11) s'étend entre :
- un bord proximal transversal situé au niveau du coude distal que forme une troisième structure en V, située sur ledit axe de symétrie, dudit premier anneau (7) ;
- des premiers bords latéraux, divergents dans la direction distale, qui s'étendent le long des segments consécutifs aux segments formant ladite deuxième structure en V ;
- des deuxièmes bords latéraux, convergeant dans la direction distale, qui s'étendent le long des segments de ladite première structure en V ; et
- le coude distal formé par ladite première structure en V ;
lesdits premier à quatrième anneaux (7) sont consécutifs les uns des autres, ou immédiatement consécutifs les uns des autres,
les bords de la membrane (9) qui délimitent ladite deuxième ouverture (11) étant successivement reliés, depuis le côté proximal et en direction du côté distal de la partie aortique (2), au coude distal formé par ladite troisième structure en V, auxdits segments consécutifs aux segments formant ladite deuxième structure en V, auxdits segments formant ladite première structure en V et audit coude distal formé par cette première structure en V.

2. Implant (1) selon la revendication 1, **caractérisé en ce que** ledit renfort (15) est radio-opaque et flexible, et **en ce que** ladite première ouverture (10) est située à proximité du coude distal de ladite première structure en V formée par ledit troisième anneau (7).

3. Implant (1) selon la revendication 1 ou la revendication 2, **caractérisé en ce que** les coudes distaux de l'un desdits premier à troisième anneaux (7) sont situés à une distance de 0 à 10 mm d'un plan transversal à la partie aortique (2) passant par les coudes proximaux de l'anneau (7) distalement adjacent.

4. Implant (1) selon l'une des revendications 1 à 3, **caractérisé en ce que** lesdits premier à quatrième anneaux présentent, à l'état déployé, un diamètre supérieur à celui que présente la membrane à l'état déployé.

5. Implant (1) selon la revendication 4, **caractérisé en ce que** les anneaux ont, à l'état déployé, un diamètre qui est de 1 à 30% supérieur au diamètre que présente la membrane à l'état déployée, et qui est de préférence de l'ordre de 20% supérieur à ce diamètre.

6. Implant (1) selon l'une des revendications 1 à 5, **caractérisé en ce que** l'extrémité de ladite branche (3) destinée à être engagée au travers de ladite première ouverture (10) présente, dans l'état de déploiement de ladite branche (3), une collerette ou une portion (3a) de forme évasée ayant un plus grand diamètre que celui de ladite première ouverture (10), cette collerette ou cette portion (3a) de forme évasée étant ainsi apte à prendre appui contre ladite partie aortique (2), au niveau de la face interne de cette dernière.

7. Implant (1) selon l'une des revendications 1 à 6, **caractérisé en ce que** ladite première ouverture (10) a un diamètre allant de 5 à 11 mm, de préférence égal à 8 mm.

8. Implant (1) selon l'une des revendications 1 à 7, **caractérisé en ce que** ladite deuxième ouverture (11) (11) a une dimension dans la direction longitudinale de ladite partie aortique (2) allant de 20 à 40 mm et une dimension dans la direction circonférentielle de cette partie aortique (2) allant de 20 à 40 mm.

9. Implant (1) selon l'une des revendications 1 à 8, **caractérisé en ce que** ladite partie aortique (2) comprend un ou plusieurs marqueurs radio-opaques (16) situés au voisinage de ladite deuxième ouverture (11), agencés de façon à permettre de visualiser le plan de cette deuxième ouverture (11) sur une image d'imagerie médicale.

10. Ensemble formé par deux implants aortiques de type stent Implant selon l'une des revendications 1 à 9, **caractérisé en ce que** cet ensemble comprend :
- un premier implant (1) dans lequel le bord de ladite première ouverture (10) tourné vers le bord adjacent de ladite deuxième ouverture (11) se trouve à une distance de ce bord adjacent allant de 4 à 8 mm, de préférence égale à 5 mm ; et
- un deuxième implant (1) dans lequel le bord de ladite première ouverture (10) tourné vers le bord adjacent de ladite deuxième ouverture (11) se trouve à une distance de ce bord adjacent allant de 8 à 12 mm, de préférence égale à 10 mm.

## Patentansprüche

1. Aorten-Implantat (1) vom Stent-Typ, umfassend einen Aortenteil (2) und einen Strang (3), der dazu bestimmt ist, in der linken Arteria subclavia platziert zu werden; wobei der Aortenteil (2) eine streckbare Armierung (5, 6, 7 ,8) aus Metalldraht und eine Membran (9), die diese Armierung bedeckt, umfasst;
wobei die streckbare Armierung Ringe (7) aufweist, die voneinander unabhängig sind und einzig mittels der Membran (9) miteinander verbunden sind; wovon jeder von einem Metalldraht als Streckenzug gebildet ist, wobei jeder Ring auf diese Weise geradlinige Segmente, die durch Winkelstücke getrennt sind, zwei aufeinander folgende Segmente und ein Winkelstück, das sich zwischen diesen Segmenten zur Bildung einer V-förmigen Struktur erstreckt, aufweisen; wobei die Armatur einen ersten, zweiten, dritten und vierten Ring (7), von dem proximalen Ende des Aortenteils (2) aus gezählt, umfasst;
wobei der Strang (3) von einer streckbaren Armierung gebildet ist, die, in einem zusammengezogenen Zustand, eingerichtet ist, um in die linke Arteria subclavia gesteckt zu werden, und, in einem gedehnten Zustand, eingerichtet ist, um sich gegen die Wand dieser Arterie abzustützen;
wobei das Implantat eine Öffnung (11), so genannte zweite Öffnung (11), aufweist, die so dimensioniert ist, dass sie sich nach dem Einsetzen des Implantats im Wesentlichen den Mündungen der Arteria brachiocephalica und der linken Arteria carotis communis zugewandt befindet;
**dadurch gekennzeichnet, dass**
der Aortenteil (2) und der Strang (3) von zwei getrennten Untereinheiten gebildet sind, die eingerichtet sind, um zusammengefügt zu werden;
der Aortenteil (2) eine erste Öffnung (10) aufweist, die durch die Membran (9) hindurch vorgesehen ist und so dimensioniert ist, dass sie eine Fläche aufweist, die wenigstens gleich oder kleiner ist als der Querschnitt, den der Strang (3) in seinem gestreckten Zustand in der linken Arteria subclavia aufweist; die zweite Öffnung (11) auf dem Aortenteil (2), durch die Membran (9) hindurch, vorgesehen ist und relativ zu der ersten Öffnung (10) so angeordnet und dimensioniert ist, dass sie sich im Wesentlichen der Mündung der linken Arteria carotis communis zugewandt befindet, wenn die erste Öffnung (10) sich im Wesentlichen der Mündung der linken Arteria subclavia zugewandt befindet;
**dadurch gekennzeichnet, dass**:
- die Mitte der ersten Öffnung (10) auf der Symmetrieachse einer ersten V-förmigen Struktur, die von dem dritten Ring (7) gebildet ist, liegt, wobei die zwei Segmente dieser ersten V-förmigen Struktur in Richtung des proximalen Endes des Aortenteils (2) divergieren; die erste Öffnung von einem Rand begrenzt ist, der eine Verstärkung (15) aufweist, die sich über den gesamten Umfang dieser ersten Öffnung (10) erstreckt;
- die zweite Öffnung (11) einen proximalen Teil umfasst, der oberhalb einer zweiten V-förmigen Struktur, die von dem zweiten Ring (7) gebildet ist, vorgesehen ist, wobei die zwei Segmente dieser zweiten Struktur in Richtung des proximalen Endes des Aortenteils (2) divergieren und diese auf der Symmetrieachse liegt; sich die zweite Öffnung (11) in der Länge erstreckt zwischen
- einem quergerichteten proximalen Rand, der sich im Bereich des distalen Kniestücks befindet, das von einer dritten V-förmigen Struktur, die auf der Symmetrieachse liegt, des ersten Rings (7) gebildet ist;
- ersten seitlichen Rändern, die in der distalen Richtung divergieren und sich entlang der Segmente erstrecken, die auf die Segmente folgen, welche die zweite V-förmige Struktur bilden;
- zweiten seitlichen Rändern, die in der distalen Richtung divergieren und sich entlang der Segmente der ersten V-förmigen Struktur erstrecken; und
- dem distalen Winkelstück, das von der ersten V-förmigen Struktur gebildet ist;
der erste bis vierte Ring (7) aufeinander folgen oder unmittelbar aufeinander folgen, wobei die Ränder der Membran (9), welche die zweite Öffnung (11) begrenzen, sukzessive, von der proximalen Seite und in Richtung der distalen Seite des Aortenteils (2), verbunden sind mit dem distalen Kniestück, das von der dritten V-förmigen Struktur gebildet ist, mit den Segmenten, die auf die Segmente folgen, welche die zweite V-förmige Struktur bilden, mit den Segmenten, welche die erste V-förmige Struktur bilden, und mit dem distalen Kniestück, das von dieser ersten V-förmigen Struktur gebildet ist.

2. Implantat nach Anspruch 1, **dadurch gekennzeichnet, dass** die Verstärkung (15) strahlenundurchlässig und flexibel ist und dass die erste Öffnung (10) sich in der Nähe des distalen Kniestücks der ersten V-förmigen Struktur befindet, die von dem dritten Ring (7) gebildet ist.

3. Implantat (1) nach Anspruch 1 oder Anspruch 2, **dadurch gekennzeichnet, dass** die distalen Kniestücke eines des ersten bis dritten Rings (7) sich in einem Abstand von 0 bis 10 mm von einer Ebene befinden, die zu dem Aortenteil (2) quergerichtet ist und durch die proximalen Kniestücke des distal angrenzenden Rings (7) verläuft.

4. Implantat (1) nach einem der Ansprüche 1 bis 3, **dadurch gekennzeichnet, dass** der erste bis vierte Ring, im gestreckten Zustand, einen Durchmesser aufweisen, der größer ist als der, den die Membran im gestreckten Zustand aufweist.

5. Implantat (1) nach Anspruch 1, **dadurch gekennzeichnet, dass** die Ringe, im gestreckten Zustand, einen Durchmesser aufweisen, der 1 bis 30 % größer ist als der Durchmesser, den die Membran im gestreckten Zustand aufweist, und der bevorzugt in der Größenordnung von 20 % größer als dieser Durchmesser ist.

6. Implantat (1) nach einem der Ansprüche 1 bis 5, **dadurch gekennzeichnet, dass** das Ende des Strangs (3), das zum Einbringen durch die erste Öffnung (10) hindurch bestimmt ist, im gestreckten Zustand des Strangs (3), einen Kragen oder einen Teil (3a) mit aufgeweiteter Form aufweist, der einen Durchmesser aufweist, der größer ist als der der ersten Öffnung (10), wobei dieser Kragen oder dieser Teil (3a) mit aufgeweiteter Form auf diese Weise eingerichtet ist, um sich gegen den Aortenteil (2), im Bereich der Innenfläche dieses letzten, abzustützen.

7. Implantat (1) nach einem der Ansprüche 1 bis 6, **dadurch gekennzeichnet, dass** die erste Öffnung (10) einen Durchmesser aufweist, der von 5 bis 10 mm beträgt, bevorzugt gleich 8 mm ist.

8. Implantat (1) nach einem der Ansprüche 1 bis 7, **dadurch gekennzeichnet, dass** die zweite Öffnung (11) (11) eine Abmessung in der Längsrichtung des Aortenteils (2) aufweist, die von 20 bis 40 mm beträgt, und eine Abmessung in der Umfangsrichtung dieses Aortenteils (2) aufweist, die von 20 bis 40 mm beträgt.

9. Implantat (1) nach einem der Ansprüche 1 bis 8, **dadurch gekennzeichnet, dass** der Aortenteil (2) eine oder mehrere strahlenundurchlässige Marker (16) umfasst, die sich in der Nähe der zweiten Öffnung (11) befinden und so eingerichtet sind, dass sie die Anzeige der Ebene dieser zweiten Öffnung (11) auf einem Bild der medizinischen Bildgebung gestatten.

10. Anordnung, gebildet aus zwei Aortenimplantaten vom Stent-Typ Implantat nach einem der Ansprüche 1 bis 9, **dadurch gekennzeichnet, dass** diese Anordnung umfasst:
- ein erstes Implantat (1), wobei der Rand der ersten Öffnung (10), der zu dem angrenzenden Rand der zweiten Öffnung (11) hin weist, sich in einem Abstand von diesem angrenzenden Rand befindet, der von 4 bis 8 mm beträgt, bevorzugt gleich 5 mm ist; und
- ein zweites Implantat (1), wobei der Rand der ersten Öffnung (10), der zu dem angrenzenden Rand der zweiten Öffnung (11) hin weist, sich in einem Abstand von diesem angrenzenden Rand befindet, der von 8 bis 12 mm beträgt, bevorzugt gleich 10 mm ist.

## Claims

1. Stent-type aortic implant (1) comprising an aortic portion (2) and a branch (3) intended to be placed in the left subclavian artery; said aortic portion (2) comprises a deployable wire frame (5, 6, 7, 8) and a membrane (9) covering this frame; **characterised in that**:
said deployable frame includes annuli (7) independent of each other and connected to each other only by means of the membrane (9), each one being formed by a broken line-shaped wire, each annulus thus having straight segments separated by bends, two consecutive segments and a bend which extends between these segments defining a V-shaped structure; the frame comprises first, second, third and fourth annuli (7) counted from the proximal end of the aortic portion (2);
said aortic portion (2) and said branch (3) are formed by two separate subassemblies that can be assembled to each other;
said branch (3) is formed by a deployable frame adapted, in a contracted state, to be engaged in the left subclavian artery and adapted, in an expanded state, to press against the lining of this artery;
said aortic portion (2) comprises a first opening (10) created through said membrane (9), dimensioned so as to have an area at least equal, or less than, the transverse cross-section of said branch (3) in its deployed state in the left subclavian artery; said aortic portion (2) further comprises a second opening (11) created through said membrane (9), arranged relative to said first opening (10), and dimensioned so as to be located substantially opposite the outlet of the left common carotid artery when said first opening (10) is located substantially opposite the outlet of the left subclavian artery;
**characterised in that**:
- the centre of said first opening (10) is located on the axis of symmetry of a first V-shaped structure formed by said third annulus (7), the two segments of this first V-shaped structure diverging towards the proximal end of said aortic portion (2); the first opening is defined by an edge provided with a reinforcement (15) which extends over the entire periphery of this first opening (10);
- said second opening (11) comprises a proximal portion arranged above a second V-shaped structure formed by said second annulus (7), the two segments of this second structure diverging towards the proximal end of the aortic portion (2) and being located on said axis of symmetry; lengthwise, said second opening (11) extends between:
- a transverse proximal edge located at the distal bend formed by a third V-shaped structure, located on said axis of symmetry, of said first annulus (7);
- first lateral edges, diverging in the distal direction, which extend along the segments consecutive to the segments forming said second V-shaped structure;
- second lateral edges, converging in the distal direction, which extend along the segments of said first V-shaped structure; and
- the distal bend formed by said first V-shaped structure;
said first to fourth annuli (7) are consecutive to each other, or immediately consecutive to each other,
said second opening (11) being dimensioned so that it is located substantially opposite the outlets of the brachiocephalic and left common carotid arteries after placing the implant;
the edges of the membrane (9) which define said second opening (11) being successively connected, from the proximal side and towards the distal side of the aortic portion (2), to the distal bend formed by said third V-shaped structure, to said segments consecutive to the segments forming said second V-shaped structure, to said segments forming said first V-shaped structure and to said distal bend formed by this first V-shaped structure.

2. Implant (1) according to claim 1, **characterised in that** said reinforcement (15) is radio-opaque and flexible, and **in that** said first opening (10) is located near the distal bend of said first V-shaped structure formed by said third annulus (7).

3. Implant (1) according to claim 1 or claim 2, **characterised in that** the distal bends of one of said first to third annuli (7) are located at a distance of 0 to 10 mm from a plane transverse to the aortic portion (2) passing through the proximal bends of the distally adjacent annulus (7).

4. Implant (1) according to one of claims 1 to 3, **characterised in that** the diameter of said first to fourth annuli, in the deployed state, is greater than that of the membrane in the deployed state.

5. Implant (1) according to claim 4, **characterised in that** the diameter of the annuli in the deployed state is 1 to 30 % greater than that of the membrane in the deployed state, and preferably about 20 % greater than this diameter.

6. Implant (1) according to one of claims 1 to 5, **characterised in that** the end of said branch (3) intended to be engaged through said first opening (10) has, in the deployed state of said branch (3), a collar or a flared portion (3a) of diameter greater than that of said first opening (10), this collar or this flared portion (3a) thus being able to press against said aortic portion (2), on the inner side of the latter.

7. Implant (1) according to one of claims 1 to 6, **characterised in that** the diameter of said first opening (10) ranges from 5 to 11 mm, and is preferably equal to 8 mm.

8. Implant (1) according to one of claims 1 to 7, **characterised in that** the dimension of said second opening (11) in the longitudinal direction of said aortic portion (2) ranges from 20 to 40 mm and in the circumferential direction of this aortic portion (2) from 20 to 40 mm.

9. Implant (1) according to one of claims 1 to 8, **characterised in that** said aortic portion (2) comprises one or more radio-opaque markers (16) located near said second opening (11), arranged so as to display the plane of this second opening (11) on a medical imaging image.

10. Assembly formed by two stent-type aortic implants according to one of claims 1 to 9, **characterised in that** this assembly comprises:
- a first implant (1) in which the edge of said first opening (10) turned towards the adjacent edge of said second opening (11) is located at a distance from this adjacent edge ranging from 4 to 8 mm, preferably equal to 5 mm; and
- a second implant (1) in which the edge of said first opening (10) turned towards the adjacent edge of said second opening (11) is located at a distance from this adjacent edge ranging from 8 to 12 mm, preferably equal to 10 mm.
